# EUROPEAN PATENT APPLICATION

(11) **EP 2 228 697 A2**
(43) Date of publication of application: **15.09.2010**
(21) Application number: 10156244.5
(22) Date of filing: 11.03.2010
(51) Int. Cl.: G05B 19/042

(54) **Domotic system for disabled persons**

(30) Priority: 11.03.2009 ES 200900679
(71) Applicant: Sanz Juez, Carlos, 28043 Madrid (ES); Zato Recellado, Jose Gabriel, 28043 Madrid (ES)
(72) Inventor: Sanz Juez, Carlos, 28043 Madrid (ES); Zato Recellado, Jose Gabriel, 28043 Madrid (ES)
(74) Representative: Pons Ariño, Angel

(57) **Abstract**

A domotic system and control process that allows a disabled user with reduced mobility to manage the basic domotic functionalities of a building, as well as providing access to a series of computer services. The system comprises a central control unit (1), a screen 2) and a domotic bus (5) which acts as a link to the devices to be controlled (6), also having non-tactile binary detection means (7) that detect a non-tactile action carried out by the disabled user and are connected to the central control unit (1) for activating one of the devices (6). Preferably, three groups of main icons are displayed on the screen (2); computer applications (8), electrical appliances (9) and furnishings and infrastructure (10), each of which contain secondary icons related to the devices to be controlled (6), each option being named through loudspeakers (4).

## Description

### OBJECT OF THE INVENTION

The present invention relates to the domotics sector for controlling domestic electrical appliances, as well as for controlling sensors and actuating alarms.

The main object of the present invention is a system that allows a building to be controlled domotically by a disabled person with reduced mobility.

### BACKGROUND OF THE INVENTION

The evolution towards quality of life is the driving force behind technologies applied to well-being in all fronts of life, even in their most basic exponents such as housing, fashion, food, etc. Most activities are carried out on closed premises (dwellings, offices, factories, recreation halls) which require a greater rationalisation of their concept, aimed at improving the use and security of the facilities for users.

This gave rise to domotics as a science aimed at achieving, by means of technology applications, an improvement in the quality of life of people.

At present, there are different domotics systems in the market applicable to buildings generally comprised of a central unit in charge of controlling the system and managing the different elements associated thereto. Said domotics systems include the necessary elements for carrying out certain functions such as: control of domestic appliances, detection of intrusion, telephone search, recording of voice messages and remote activation/deactivation of system functions.

The aforementioned systems are aimed at people with medium/high knowledge of new technologies, the use and understanding of which are very complicated for a disabled person.

### DESCRIPTION OF THE INVENTION

The domotic control system of the present invention allows a disabled user with reduced mobility to manage the basic domotic functionalities of a building, in addition to providing access to a series of computer services.

Said system enables a physically disabled and highly dependent person to control the following devices: television, radio, Internet, telephone calls, videoconference, games, virtual keyboard and mouse, etc. Likewise, it enables the domotic control of a building's furnishing elements such as: doors, blinds, lights, temperature, closets, beds, tables with adjustable height, etc. The system has been developed in such a manner that, with the minimum effort, a disabled user can control his/her entire house, from switching on the television set in the living room to switching on the coffee machine.

The system is mainly comprised of a central control unit, a screen and a domotic bus which acts as a link to the devices to be controlled, also having non-tactile binary detection means that detect a non-tactile action carried out by the disabled user and are connected to the central control unit for activating one of the devices.

Preferably, the system comprises tactile activation means such as a mouse, keyboard, etc. for user-machine interaction. The system may also have loudspeakers.

The system is designed so that the incoming signal is of the binary type, allowing the user to choose between "yes/no" (1,0) by means of a system that detects blinking, blowing, a head movement, a push-button signal or any other binary input technique for communicating with disabled persons.

With regard to the control process, the system that is the object of the invention comprises the following stages:
- on-screen display of a series of main icons related to the devices to be controlled, wherein the presentation is made using an icon sweep, which consists of the individual and consecutive appearance of each icon on the screen;
- detection of the user's non-tactile action by the non-tactile binary detection means upon displaying the preferred main icon and sending of a signal to the control unit, activation of the main icon that motivated the user's action and sending of an activation signal from the control unit to the device to be controlled by the domotic bus.

In a preferred embodiment of the invention, the system has three groups of main icons: computer applications, electrical appliances and furnishings and infrastructure.

Activation of the corresponding main icon determines the on-screen display of secondary icons directly associated with the devices to be controlled; for example, the television set is associated with the image of a television set.

Preferably, the background colour of each image displayed on the screen changes and each option is named through loudspeakers. When the sweep ends it starts again.

In a preferred embodiment, when the programme is run it appears in the first group by default and in order to switch to another group we simply have to wait for the sweep to pass the first icon on the left, which is the icon that links to the other groups.

Likewise, in another preferred embodiment of the invention, the system additionally comprises a group-based activity association tool that arranges the activities in order. Thus, the icons corresponding to the most frequent actions are shown in the first group.

### DESCRIPTION OF THE DRAWINGS

In order to complement this description and for the purpose of helping to better understand the characteristics of the invention, in accordance with a preferred example of practical embodiment thereof, a set of drawings has been included as an integral part of said description, wherein the following have been represented in an illustrative and non-limiting manner:
Figure 1 shows a block diagram of the domotic control system that is the object of the invention.
Figure 2 shows a sequence of stages of the control process that is the object of the invention.

### PREFERRED EMBODIMENT OF THE INVENTION

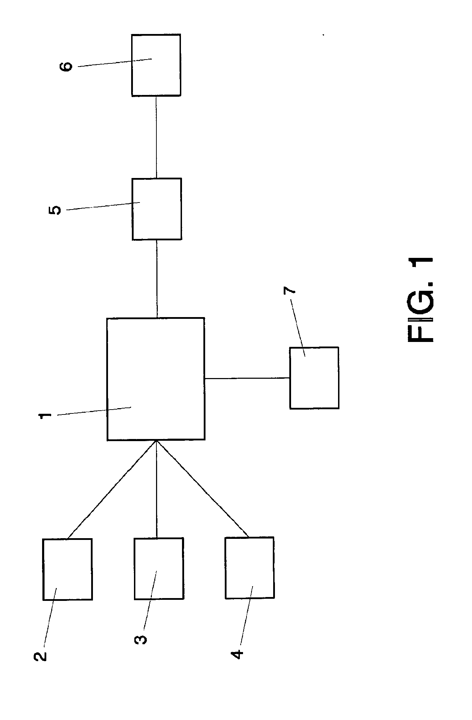

As we can observe in figure 1, the system is mainly comprised of a central control unit (1), a screen (2) and a domotic bus (5) that acts as a link to the devices (6) to be controlled, also having non-tactile binary detection means (7) that detect a non-tactile action carried out by the disabled user and are connected to the central control unit (1) for activating one of the devices (6).

Preferably, the system comprises tactile activation means (3) such as a mouse, keyboard, etc. for user-machine interaction. The system may also have loudspeakers (4).

The system is designed so that the incoming signal is of the binary type, allowing the user to choose between "yes/no" (1,0) by means of a system that detects blinking, blowing, a head movement, a push-button signal or any other binary input technique for communicating with disabled people.

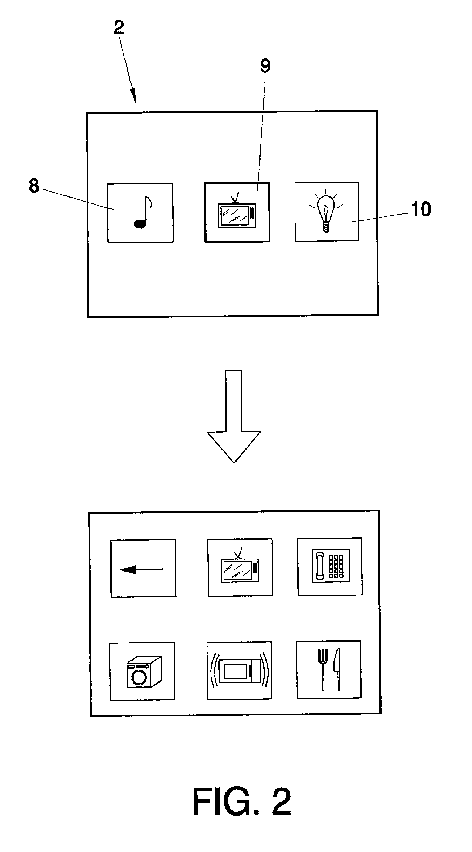

Figure 2 shows a sequence of stages of the control process that comprises the following stages:
- on-screen (2) display of a series of main icons related to the devices to be controlled (6), presented using an icon sweep, whereby each icon appears individually and consecutively on the screen (2);
- detection of the user's non-tactile action by the non-tactile binary detection means (7) upon displaying the preferred main icon and sending of a signal to the control unit (1), activation of the main icon that motivated the user's action and sending of an activation signal from the control unit (1) to the device to be controlled (6) by the domotic bus (5).

In a preferred embodiment of the invention, the system has three groups of main icons: computer applications (8), electrical appliances (9) and furnishings and infrastructure (10).

Activation of the corresponding main icon determines the on-screen (2) display of secondary icons directly associated with the devices to be controlled (6).

Preferably, the background colour of each image displayed on the screen (2) changes and each option is named through loudspeakers (4). When the sweep ends it starts again.

In a preferred embodiment, when the programme is run it appears in the first group by default and in order to switch to another group we simply have to wait for the sweep to pass the first icon on the left, which is the icon that links to the other groups.

Likewise, in another preferred embodiment of the invention, the system also comprises a group-based activity association tool that allows the activities to be arranged in order. Thus, in the first group the icons corresponding to the most frequent actions are shown.

## Claims

1. Domotic control system for a dwelling destined for disabled users with reduced mobility aimed at facilitating the management of the basic domotic functionalities of a dwelling, as well as providing access to a series of computer services, comprising a central control unit (1), a screen (2) and a domotic bus (5) which acts as a link to the devices to be controlled (6), **characterised in that** it also comprises non-tactile binary detection means (7) that detect a non-tactile action carried out by the disabled user and are connected to the central control unit (1) for activating one of the devices (6).

2. Domotic control system for a dwelling, according to claim 1, **characterised in that** it also comprises tactile activation means (3).

3. Domotic control system for a dwelling, according to any one of claims 1 or 2, **characterised in that** it also comprises loudspeakers (4).

4. Domotic control system for a dwelling, according to claim 1, **characterised in that** the non-tactile binary detection means (7) are selected from among means for detecting blinking, blowing, user head movements and blowing detection means.

5. Control process for the system described in claim 1, **characterised in that** it comprises the following stages:
- on-screen (2) display of a series of main icons related to the devices to be controlled (6), presented by means of an icon sweep wherein each icon appears individually and consecutively on the screen (2);
- detection of the user's non-tactile action by the non-tactile binary detection means (7) upon displaying the preferred main icon and sending of a signal to the control unit (1), activation of the main icon that motivated the user's action and sending of an activation signal from the control unit (1) to the device to be controlled (6) by the domotic bus (5).

6. Process, according to claim 5, **characterised in that** the main icons comprise: computer applications (8), electrical appliances (9) and furnishings and infrastructure (10), wherein each contains images related to the devices to be controlled (6).

7. Process, according to claim 5, **characterised in that** the activation of the main icon determines the display on the screen (2) of secondary icons directly associated with the devices to be controlled (6).

8. Process, according to any one of claims 5 to 7, **characterised in that** the display of the icons entails an associated change in the background colour of the screen (2).

9. Process, according to any one of claims 5 to 8, **characterised in that** the display of the icons entails naming the device to be controlled (6) through loudspeakers (4).
